# EUROPEAN PATENT APPLICATION

(11) **EP 1 491 892 A1**
(43) Date of publication of application: **29.12.2004**
(21) Application number: 03745933.6
(22) Date of filing: 07.04.2003
(51) Int. Cl.: G01N 33/543

(54) **TEST PIECE FOR CHROMATOGRAPHY AND PROCESS FOR PRODUCING THE SAME**

(30) Priority: 05.04.2002 JP 2002103281
(71) Applicant: Matsushita Electric Industrial Co., Ltd., Kadoma-shi, Osaka 571-8501 (JP)
(72) Inventor: YUGAWA, Keiko, Nara-shi, Nara 631-0041 (JP); KITAWAKI, Fumihisa, Katano-shi, Osaka 576-0021 (JP); KAWAMURA, Tatsurou, Kyotanabe-shi, Kyoto 610-0351 (JP)
(74) Representative: Grünecker, Kinkeldey, Stockmair & Schwanhäusser Anwaltssozietät
(86) International application number: PCT/JP2003/004377
(87) International publication number: WO 2003/085402

(57) **Abstract**

As shown in FIG. **1**, an immunochromatography test strip **10** of the present embodiment is made of a base material **11** which includes a sample introduction section **12**, a labeling section **13** and an immobilization section **14**. The base material **11** is made of a material that is capable of being impregnated with a solvent of a sample solution containing a detection target substance. The material allows migration of a solvent of a sample solution by capillary action. The sample introduction section **12** is a region where a sample solution containing a detection target substance is introduced (e.g., dripped). The sample introduction section **12** contains a metal salt supported thereon. The labeling section **13** contains a labeled antibody **15**, which is labeled with a labeling substance, in a state such that the labeled antibody **15** can be eluted into the sample solution. The labeled antibody **15** used herein specifically binds to a detection target substance. The immobilization section **14** contains an immobilized antibody **16**. The immobilized antibody **16** used herein specifically binds to the detection target substance.

## Description

### Technical Field

The present invention relates to a test strip for chromatography with which a particular component in a sample solution is quickly and precisely quantized in a convenient manner.

### Background Art

Conventionally, dry chemistry has been proposed as a methodology for conveniently quantizing a particular component in a sample solution without diluting or stirring the sample solution.

Dry chemistry is a methodology wherein a sample solution is brought into a contact at a point with dried reagent stored in a solid phase matrix, such as a film or test paper, in order to measure a detection target substance in the sample solution.

Devices used for dry chemistry includes a single-layered device wherein a reagent is supported by a filter paper and a multi-layered device including a migration layer, a reaction layer, a reagent layer, etc. In either of these devices, it is not necessary to prepare a reagent because the reagent is already supported on the solid phase matrix. Thus, such a device can be stored in a small space. Moreover, these devices require only a small quantity of sample solution.

A representative dry chemistry assay method is immunochromatography, which utilizes an antigen-antibody reaction and capillary action (see, for example, Japanese Unexamined Patent Publication No. 10-73592). An exemplary device used in immunochromatography is a test strip wherein an immobilized antibody and an antibody labeled with a labeling substance (hereinafter, simply referred to as "labeled antibody"), which are in a dried state, are supported on a solid phase matrix made of a material that is capable of being impregnated with a solvent of a sample solution. A typical example of such a material is a membrane filter.

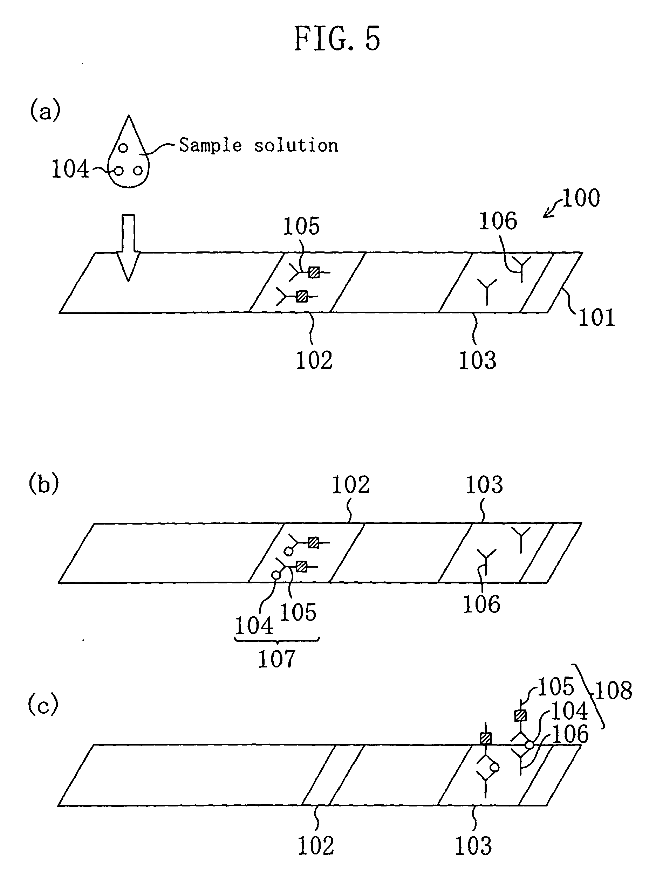

In a measurement, after a sample solution containing a detection target substance **104** is introduced into a test strip at an end of the strip as shown in FIG. **5(a),** the sample solution reaches a labeling section **102** by capillary action as shown in FIG. **5(b)**, in which a labeling antibody **105** is contained in a state such that the labeling antibody **105** can be eluted into the sample solution. In the labeling section **102**, a complex **107** of the labeling antibody **105** and the detection target substance **104** is formed. Furthermore, as shown in FIG. **5(c)**, the complex **107** migrates along with a flow of the sample solution by capillary action to an immobilization section **103** supporting an immobilized antibody **106**. In the immobilization section **103**, the complex **107** is caught by the immobilized antibody **106** through an antigen-antibody reaction. The other components of the sample solution pass through the immobilization section **103** along with the flow of the solvent by capillary action, so that only complex **108** is left in the immobilization section **103**. Herein, detection of the detection target substance **104** and measurement of the concentration of the detection target substance **104** are performed by measuring an output derived from the labeling substance **105**. Some immunochromatography methods utilize a sandwich-type antigen-antibody reaction, whereas other immunochromatography methods use a competitive antigen-antibody reaction. However, the structure of the test strip and the measurement method are the same in both methods.

The measurement method that utilizes an immunochromatography method has various advantages, such as convenience of operation, quick determination, and reduction in measurement cost, in addition to the above-described advantages of dry chemistry. Thus, such a measurement method is applicable not only to conventional clinical assays but also to Point-Of-Care (POC) assays which have been receiving attention in recent years. Note that POC is a generic name for clinical assays in medical practices wherein shortening of the time period between sampling of a specimen and obtainment of an assay result is the most significant factor.

### Problems to be solved

In the case where the above-described immunochromatography method is used for detecting a detection target substance or measuring the concentration of the detection target substance in actual medical practices, a part of blood collected using a needled syringe and contained in a blood tube is used as a sample solution.

Blood tubes contain various reagents according to the purposes. For example, a blood tube for measuring blood sugar, aldosterone, or prostaglandin E2 contains EDTA, or the like, as a chelating agent. EGTA also provides a chelating effect and thus may be contained in a blood tube. Moreover, carboxylic acids, such as citric acid, phthalic acid, oxalic acid, or the like, also exhibit a chelating effect under certain conditions. Especially, citric acid is contained in a test tube in many cases.

In the case where the detection target substance is a protein and the structure of the protein contains a divalent metal ion, such as a calcium ion, or the like, if a substance having the chelating effect is contained in a sample solution, the substance traps the divalent metal ion, i.e., the divalent metal ion is released from the structure of the protein (detection target substance). As a result, the three-dimensional structure of the protein (detection target substance) is changed in some cases.

For example, CRP is a pentamer consisting of five subunits. The four out of the five subunits contain calcium ions. If the pentamer is present in a solution together with EDTA having a chelating effect, or the like, the calcium ions are released from the CRP, and the three-dimensional structure of the CRP changes. Also in the case where the conditions for storing collected blood are undesirable, calcium ions fall from the CRP, and the three-dimensional structure of the CRP changes in some cases.

The immunochromatography method uses an antibody that binds to a detection target substance containing a divalent metal ion. Therefore, the antibody does not bind to a detection target substance having a modified structure, or the affinity between the detection target substance and the antibody sometimes changes. Thus, there is a possibility that a correct result is not obtained.

The present invention was conceived in view of the above circumstances. An objective of the present invention is to provide a test strip for chromatography with which a detection target substance in a sample solution is precisely measured.

### Disclosure of Invention

A chromatography test strip of the present invention is a chromatography test strip for measuring a detection target substance contained in a sample solution, the chromatography test strip comprising a base material, wherein: the base material includes a sample introduction section, a labeling section and an immobilization section which are arranged such that the sample solution introduced into the sample introduction section migrates through the labeling section to the immobilization section; and a metal salt is contained in at least any of the sample introduction section, the labeling section, and an area between the sample introduction section and the labeling section.

According to the present invention, a sample solution comes into contact with a metal salt before the sample solution comes into contact with a labeling section even when a detection target substance contains a metal ion, and the detection target substance comes into contact with a substance having a chelating effect to release a metal ion so that the three-dimensional structure of the detection target substance is changed. As a result, the detection target substance takes in a metal ion and recovers its original three-dimensional structure. Thus, even when the sample solution is handled using a blood tube, or the like, which contains a substance having a chelating effect, the detection target substance in the sample solution can be precisely measured and detected.

The metal salt may exist on the surface of the base material or may exist inside the base material.

The labeling section may include a first binding substance which specifically binds to the detection target substance, the first binding substance being labeled with a labeling substance. The immobilization section may include a second binding substance which specifically binds to the detection target substance.

The first binding substance and the second binding substance are antibodies that specifically bind to the detection target substance.

With such structures, measurement of the detection target substance by immunochromatography is performed.

The metal salt may be a salt of a divalent metal ion.

The divalent metal ion may be calcium ion.

The first binding substance and the second binding substance may be an anti-CRP antibody.

The metal salt may be calcium chloride.

Another chromatography test strip of the present invention is a chromatography test strip for measuring a detection target substance contained in a sample solution, comprising: a base material; a sample introduction layer provided above the base material, the sample introduction layer containing a metal salt; a labeling layer provided on the base material so as to be in contact with the sample introduction layer, the labeling layer containing a first binding substance which specifically binds to the detection target substance in a state such that the first binding substance can be eluted into the sample solution, the first binding substance being labeled with a labeling substance; and an immobilization layer provided on the base material so as to be in contact with the labeling layer, the immobilization layer containing a second binding substance which specifically binds to the detection target substance in a state such that the second binding substance is immobilized.

According to the present invention, a sample solution comes into contact with a metal salt before the sample solution comes into contact with a labeling section even when a detection target substance contains a metal ion, and the detection target substance comes into contact with a substance having a chelating effect to release a metal ion so that the three-dimensional structure of the detection target substance is changed. As a result, the detection target substance takes in a metal ion and recovers its original three-dimensional structure. Thus, even when the sample solution is handled using a blood tube, or the like, which contains a substance having a chelating effect, the detection target substance in the sample solution can be precisely measured and detected. Furthermore, according to the present invention, the sample introduction layer is provided so as to be in contact with the labeling layer, and accordingly, the quantity of a solvent of the sample solution which is lost during migration of the sample solution is decreased. Thus, it is possible to supply a sufficient quantity of sample solution to the labeling layer and the immobilization layer.

The chromatography test strip may further comprise an absorption layer provided above the base material so as to be in contact with the immobilization layer for absorbing the sample solution.

A production method of a chromatography test strip of the present invention is a production method of a chromatography test strip used for measuring a detection target substance contained in a sample solution, comprising the steps of: (a) preparing a base sheet; (b) forming a metal salt supporting region, a labeled antibody region, an immobilized antibody region such that these regions extend in parallel to each other and the labeled antibody region is provided between the metal salt supporting region and the immobilized antibody region; and (c) cutting the base sheet in a direction perpendicular to the metal salt supporting region, the labeled antibody region and the immobilized antibody region, thereby obtaining base sheet strips, wherein the metal salt supporting region includes a metal salt supported thereon, the labeled antibody region includes a first binding substance which specifically binds to the detection target substance in a state such that the first binding substance can be eluted into the sample solution, the first binding substance being labeled with a labeling substance; and the immobilized antibody region includes a second binding substance which specifically binds to the detection target substance in a state such that the second binding substance is immobilized.

With such a method, a chromatography test strip with which a detection target substance in a sample solution is correctly measured is produced.

### Brief Description of Drawings

FIG. **1** is a schematic view showing a test strip for immunochromatography.
FIGS. **2(a)** through **2(c)** schematically illustrate a principle of a measurement that uses the test strip for immunochromatography.
FIGS. **3(a)** through **3(c)** illustrate production steps of the test strip for immunochromatography.
FIG. **4(a)** is a plan view showing a test strip for immunochromatography. FIG. **4(b)** is a cross-sectional view taken along line X-X of FIG. **4(a)**.
FIG. **5** schematically illustrates a principle of a measurement that uses a conventional test strip for immunochromatography.

### Best Mode for Carrying Out the Invention

Hereinafter, embodiments of the present invention are described with reference to the drawings.

### (Embodiment 1)

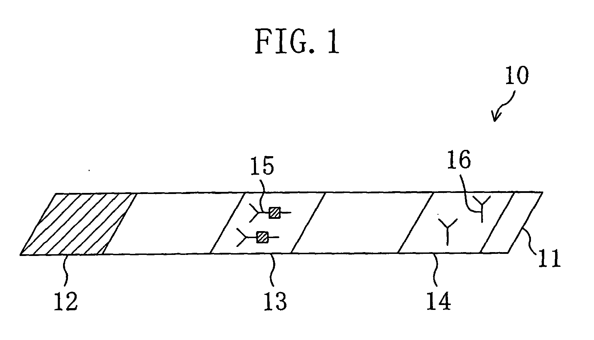

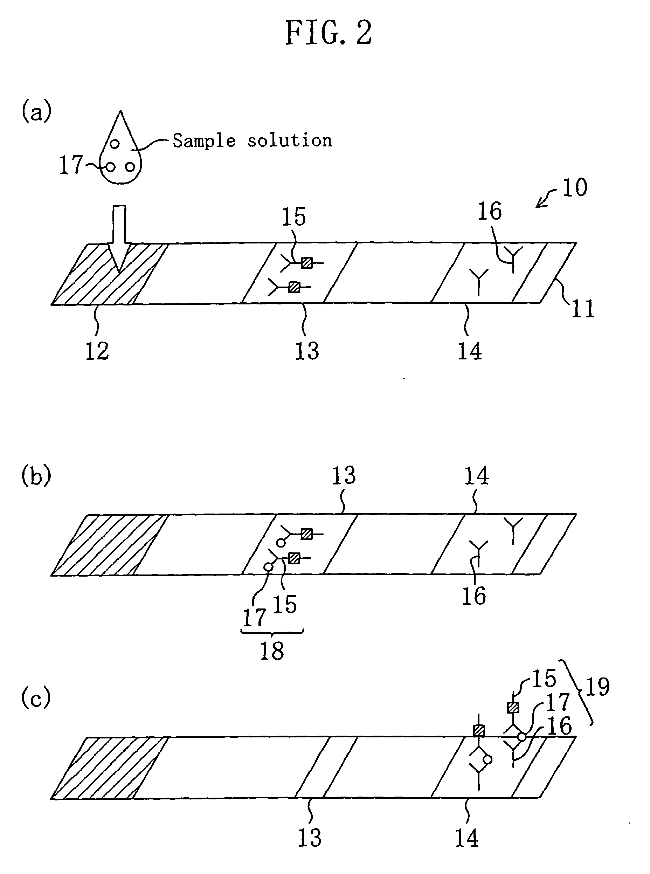

In embodiment 1, a test strip for immunochromatography (hereinafter, "immunochromatography test strip") is described with reference to the drawings. FIG. **1** shows an immunochromatography test strip according to embodiment 1. FIGS. **2(a)** through **2(c)** schematically illustrate an operation wherein the immunochromatography test strip of embodiment **1** is used.

As shown in FIG. **1,** the immunochromatography test strip **10** of embodiment 1 is made of a base material **11** which includes a sample introduction section **12,** a labeling section **13** and an immobilization section **14.**

The base material **11** is made of a material that is capable of being impregnated with a solvent of a sample solution containing a detection target substance. For example, the base material **11** is made of a porous material, such as a nitrocellulose membrane, a cellulose acetate membrane, glass fiber filter paper, nonwoven fabric, or the like. The nitrocellulose membrane is especially preferable. These materials allow migration of a solvent of a sample solution by capillary action.

The sample introduction section **12** is a region where a sample solution containing a detection target substance is introduced (e.g., dripped). The sample introduction section **12** contains a metal salt supported thereon.

The labeling section **13** contains a labeled antibody **15**, which is labeled with a labeling substance, in a state such that the labeled antibody **15** can be eluted into the sample solution. The labeled antibody **15** used herein specifically binds to a detection target substance.

The immobilization section **14** contains an immobilized antibody **16**. The immobilized antibody **16** used herein specifically binds to the detection target substance.

The metal salt supported by the sample introduction section **12** is selected according to the detection target substance. For example, if the detection target substance is CRP containing a calcium ion, a calcium salt is used such that a calcium ion is supplied to maintain the three-dimensional structure of the CRP. As a matter of course, in this case, the labeled antibody **1**5 and the immobilized antibody **16** contained in the labeling section **13** and the immobilization section **14**, respectively, are an anti-CRP antibody containing a calcium ion.

In this way, the metal salt, the labeled antibody **15** and the immobilized antibody **16** are selected according to the detection target substance.

In the measurement of a detection target substance, the following phenomenon occurs in the immunochromatography test strip **10** of embodiment 1.

In the first place, as shown in FIG. **2(a)**, the immunochromatography test strip **10** is prepared, and a sample solution containing a detection target substance **17** is dripped on the sample introduction section **12** which is provided at an end of the immunochromatography test strip **10**. Then, the solvent of the sample solution migrates from the sample introduction section **12** toward the immobilization section **14.** As a result, as shown in FIG. **2(b)**, the sample solution reaches the labeling section **13** by capillary action, and a complex **18** of the labeled antibody **15** and the detection target substance **17** is formed through an antigen-antibody reaction. The formed complex **18** migrates along a flow of the sample solution by capillary action and reaches the immobilization section **14** including the immobilized antibody **16**. In the immobilization section **14**, the complex **18** is caught by the immobilized antibody **16** to form a complex **19** as shown in FIG. **2(c)**. The components of the sample solution other than the complex **18** pass through the immobilization section **14** along with the flow of the solvent by capillary action, so that only the complex **19** is left in the immobilization section **14**. Herein, detection of the detection target substance **17** in the sample solution and measurement of the concentration of the detection target substance **17** in the sample solution can be performed by measuring an output derived from the labeling substance **15** (e.g., reflection absorbance).

According especially to the immunochromatography test strip **10** of embodiment 1, the sample solution comes into contact with a metal salt immobilized on the immunochromatography test strip **10** before the sample solution comes into contact with the labeled antibody **15** contained in the labeling section **13** even when the detection target substance **17** contains a metal ion, and the detection target substance **17** comes into contact with a substance having a chelating effect in the blood tube to release a metal ion so that the three-dimensional structure of the detection target substance **17** is changed. As a result, the detection target substance **17** takes in a metal ion and recovers its original three-dimensional structure. Thus, even when the sample solution is handled using a blood tube, or the like, which contains a substance having a chelating effect, the detection target substance **17** in the sample solution can be precisely measured and detected.

In the immunochromatography test strip **10** of embodiment 1, the labeling section **13** contains the labeled antibody **15**, which has been labeled with a labeling substance, in a state such that the labeled antibody **15** can be eluted into the sample solution. A specific example of the labeled antibody **15** is an antibody labeled with gold colloid, but the present invention is not limited thereto. For example, in an alternative test strip of the present invention, an enzyme labeled with a labeling substance, a receptor, a nucleotide labeled with a labeling substance having a specific sequence, such as a DNA, or the like, is contained in the labeling section **13** in place of the labeled antibody **15** such that it can be eluted into the sample solution.

The metal salt is only required to be supported on at least any one of the sample introduction section **12**, an area between the sample introduction section **12** and the labeling section **13,** and the labeling section **13.** Preferably, the metal salt is supported on the sample introduction section **12** or at a position in the vicinity of the sample introduction section **12** because, in such a case, the reaction duration of the detection target substance **17** and the metal salt is increased, and the effect of recovering the three-dimensional structure of the detection target substance **17** is enhanced.

In the immunochromatography test strip **10** of embodiment 1, a protein containing a metal salt in its structure can be measured and detected, although the detection target substance **17** is not limited to any particular substance. Examples of the detection target substance **17** include CRP, troponin, calmodulin, parvalbumin, etc.

In embodiment 1, a metal ion that constitutes the metal salt is not limited to the metal ion included in the detection target substance **17**. Examples of the metal ion include a calcium ion, a strontium ion, a manganese ion, a magnesium ion, etc.

It is preferable that the metal ion that constitutes the metal salt is the same as the metal ion included in the detection target substance **17** because, in such a case, the affinity between the detection target substance **17** and the labeled antibody **15** and the affinity between the detection target substance **17** and the immobilized antibody **16** do not decrease.

It should be noted that the metal salt is not limited to any particular metal salt so long as it is soluble in the sample solution. Especially, a halide salt, a nitrate and a sulfate are preferable as the metal salt because they have high solubility so that they are readily supported by the immunochromatography test strip **10** of embodiment 1, and a metal ion is readily eluted into the sample solution.

In the case where the detection target substance **17** is CRP which is a marker for infectious diseases, the metal ion that constitutes a salt of a divalent metal ion is preferably a calcium ion because CRP contains a calcium ion in its structure. Especially, it is more preferable that the salt of the divalent metal ion is calcium chloride because it has high solubility and is less expensive.

The sample solution may be any of an aqueous solution and an organic solution. Examples of the sample solution include bodily fluids, river water, seawater, groundwater, an aqueous solution in which soil or food is dissolved, etc. Examples of the bodily fluids include blood, blood plasma, serum, urine, saliva, sweat, and tear (lacrimal fluid), etc. Among these, blood is preferable as the sample solution.

According to embodiment 1, the amount of the metal salt supported on the immunochromatography test strip **10** may be adjusted according to the concentration of a chelating agent that can be contained in the sample solution. In the case where blood is obtained from a blood tube as the sample solution, the EDTA concentration in the blood obtained from the blood tube is generally in the range of 3 mM to 10 mM. Thus, it is preferable that the metal salt is supported such that a metal ion is supplied to the amount of 3 mM to 10 mM. Specifically, the molarity of calcium ion is equal to or higher than the molarity of EDTA.

The labeled antibody **15** and the immobilized antibody **16** may be any of a monoclonal antibody and a polyclonal antibody. However, it should be noted that if a monoclonal antibody is used, steric hindrance should be avoided in the process of combining the labeled antibody **15** and the immobilized antibody **16** through the detection target substance **17**.

Examples of the labeling substance for the labeled antibody **15** include a coloring substance, a fluorescent substance, a phosphorescent substance, a light-emitting substance, an oxygen-reducing substance, an enzyme, a nucleic acid, an endoplasmic reticulum, etc. Preferable examples of the coloring substance include gold colloid, silver colloid, selenium colloid, coloring latex, cyanine, an azo dye, etc. Among these, gold colloid is especially preferable.

Examples of the fluorescent substance include an aromatic compound, such as pyrene, an aromatic compound having a substituted functional group, such as dansyl, fluorescein, rhodamine, coumarin, etc. An example of the phosphorescent substance is benzophenone. An example of the light-emitting substance is a substance that causes a light-emitting reaction of luciferin and ATP. An example of the oxygen-reducing substance is a substance that causes an oxygen-reducing reaction of glucose and glucose oxidase to produce an electric current. Examples of the endoplasmic reticulum include micelle, liposome, etc.

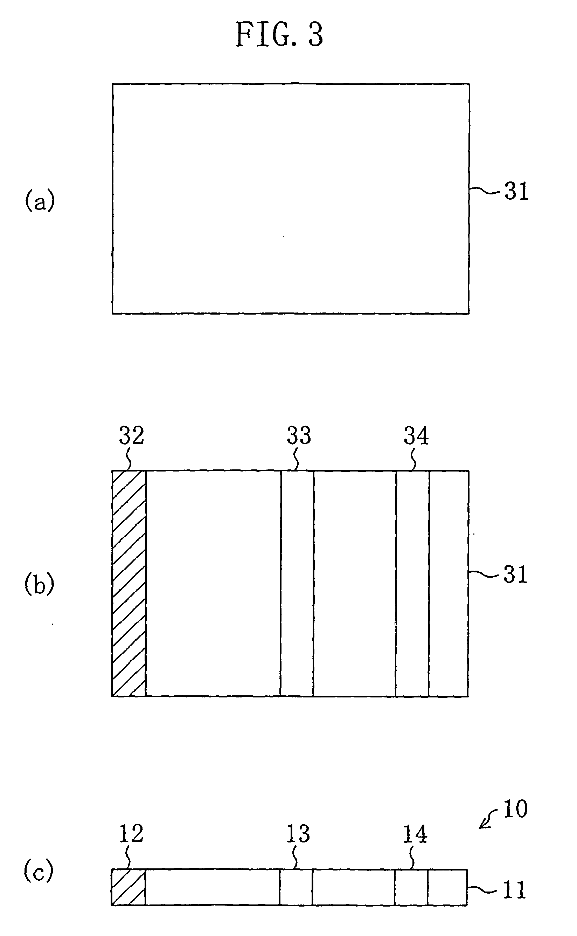

Next, a method for producing the immunochromatography test strip of embodiment 1 is described with reference to FIGS. **3(a)** and **3(c)**.

In the first place, a base sheet **31** is prepared as shown in FIG. **3(a)**. The base sheet **31** is made of the same material as that of the base material **11**, i.e., a porous material, such as a nitrocellulose membrane, a cellulose acetate membrane, glass fiber filter paper, nonwoven fabric, or the like.

Next, a metal salt supporting region **32**, a labeled antibody region **33**, and an immobilized antibody region **34** are formed in the base sheet **31** as shown in FIG. **3(b)**. The metal salt supporting region **32** is formed by impregnating the base sheet **31** with a solution that contains a metal salt dissolved therein and drying the impregnated base sheet **31**. The labeled antibody region **33** is formed by providing the base sheet **31** with an antibody labeled with a labeling substance in a state such that the labeled antibody can be eluted into a sample solution. The immobilized antibody region **34** is formed by immobilizing an antibody. As a matter of course, the labeled antibody and the immobilized antibody used herein specifically bind to a detection target substance. The metal salt supporting region **32,** the labeled antibody region **33,** and the immobilized antibody region **34** are formed so as to extend in parallel to each other. The labeled antibody region **33** is formed between the metal salt supporting region **32** and the immobilized antibody region **34**.

Then, the base sheet **31** is cut along a direction perpendicular to the metal salt supporting region **32**, the labeled antibody region **33**, and the immobilized antibody region **34** into strips, whereby an immunochromatography test strip **10** of embodiment **1** is obtained as shown in FIG. **3(c)**.

### (Embodiment 2)

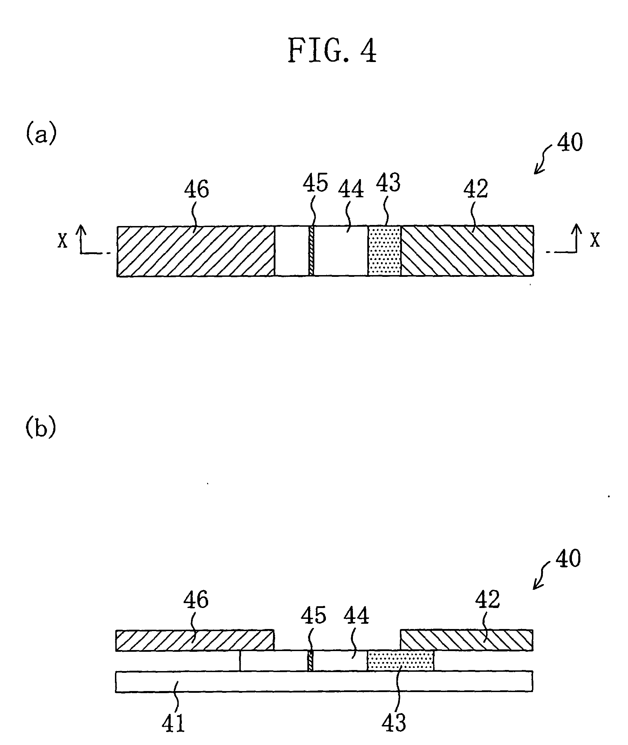

In embodiment 2, a multi-layered immunochromatography test strip is described, whereas the single-layered immunochromatography test strip has been described in embodiment 1. FIG. **4(a)** is a plan view showing an immunochromatography test strip of embodiment 2. FIG. **4(b)** is a cross-sectional view taken along line **X-X** of FIG. **4(a).**

As shown in FIGS. **4(a)** and **4(b),** the immunochromatography test strip **40** of embodiment 2 includes a base material **41,** a labeling layer **43** and an immobilization layer **44** provided on the base material **41**, a sample introduction layer **42** provided above the base material **41** so as to be in contact with the labeling layer **43**, and an absorption layer **46** provided above the base material **41** so as to be in contact with the immobilization layer **44**.

The material of the base material **41** is not limited to any particular material but is preferably a material that prevents a solvent of a sample solution containing a detection target substance from passing therethrough. Specifically, a resin such as polyethylene terephthalate, or the like, is used.

The sample introduction layer **42**, the labeling layer **43**, the immobilization layer **44**, and the absorption layer **46** are each made of a material that is capable of being impregnated with a sample solution containing a detection target substance. For example, these layers are made of a porous material, such as a nitrocellulose membrane, a cellulose acetate membrane, glass fiber filter paper, nonwoven fabric, or the like. The nitrocellulose membrane is especially preferable. These materials allow a solvent of a sample solution to migrate by capillary action. It should be noted that in embodiment 2, the sample introduction layer **42**, the labeling layer **43**, the immobilization layer **44**, and the absorption layer **46** are made of nonwoven fabric, a labeling filter paper available from Whatman Corporation, a nitrocellulose membrane, and glass fiber filter paper, respectively.

The sample introduction layer **42** is a region where a sample solution containing a detection target substance is introduced (e.g., dripped). The sample introduction layer **42** includes a metal salt supported thereon.

The labeling layer **43** contains the labeled antibody, which has been labeled with a labeling substance, in a state such that the labeled antibody can be eluted into the sample solution. The labeled antibody (not shown) specifically binds to the detection target substance.

The immobilization layer **44** includes an immobilized antibody **45**. The immobilized antibody **45** specifically binds to the detection target substance.

The absorption layer **46** absorbs the sample solution from the immobilization layer **44**.

In the multi-layered immunochromatography test strip **40** of embodiment 2, the sample introduction layer **42** is provided so as to be in contact with the labeling layer **43**, and accordingly, the quantity of a solvent of the sample solution which is lost during migration of the sample solution is decreased. Thus, it is possible to supply a sufficient quantity of sample solution to the labeling layer **43** and the immobilization layer **44**.

Since the absorption layer **46** for absorbing the sample solution is provided on the immobilization layer **44**, an excessive portion of the sample solution contained in the labeling layer **43** and the immobilization layer **44** is absorbed by the absorption layer **46**. The absorption layer **46** is provided in embodiment 2 but may be omitted as necessary.

Alternatively, the labeling layer **43** may also serve as the sample introduction layer **42.** In such a case, the sample solution is brought into direct contact at a point with the labeling layer **43**, whereby the sample solution is introduced into the immunochromatography test strip **40**.

### EXAMPLES

Hereinafter, specific examples are shown in order to describe the present invention in more detail.

### (Example 1)

In example 1, the immunochromatography test strip **10** of embodiment 1 was used to perform measurement of a detection target substance. It should be noted that the detection target substance in the sample solution was detected by visually confirming the labeled substance **15** left in the immobilization section **14**.

### --- Preparation of immunochromatography test strip ---

A base material **11** made of a nitrocellulose membrane (width: 5 mm, length: 50 mm) was immersed into 10 mM calcium chloride solution and taken out therefrom as it was. The base material **11** was dried until the water content completely evaporated, thereby forming a sample introduction section **12**. Then, anti-CRP serum (produced by Cosmo Bio Co., Ltd.; immune animal: goat) was affinity-purified and sensitized with gold colloid particles (labeling substance). The resultant anti-CRP serum (5 mg/ml) was employed as the labeled antibody **15**. Specifically, 500 µl of the resultant anti-CRP serum was provided so as to be supported on the dried base material **11**, thereby forming the labeling section **13**. Then, 200 ml of anti-CRP monoclonal antibody (2.5 mg/ml: O-CRP-MCA produced by Oriental Yeast, Co., Ltd.) was dripped onto a nitrocellulose membrane and dried, thereby producing the nitrocellulose membrane on which the anti-CRP monoclonal antibody was adsorbed as the immobilized antibody **16**. The resultant nitrocellulose membrane was attached onto the base material **11** to form an immobilization section **14**, whereby the immunochromatography test strip **10** shown in FIG. **1** was obtained.

The produced immunochromatography test strip **10** was used in the following assay. First, 1 ml of control blood (CRP: 1 mg/dl) was collected into a blood tube used for measurement of blood sugar, HbA1 and HbA1c (produced by FALCO biosystems Ltd.) which contained 3.7 mg of EDTA-2Na. Then, 50 µl of the collected blood was introduced to the sample introduction section **12** as a sample solution and incubated at room temperature for 5 minutes.

By visual observation, the color of magenta derived from the gold colloid was found in the immobilization section **14**. From this, the CRP (detection target substance) in the sample solution was detected.

In this example, blood samples containing CRP at different concentrations were introduced to the immunochromatography test strips **10**, and the labeling substance in the immobilization section **14** was measured using a densitometer. As a result, it was confirmed that the CRP concentration in the blood was proportional to the concentration of the labeling substance.

### (Comparative Example 1)

In comparative example 1, an immunochromatography test strip was produced in the same way as described in example 1, except that a base material is not immersed into calcium chloride solution. Then, as in example 1, 50 µl of the control blood (CRP: 1 mg/dl) was introduced to the resultant immunochromatography test strip.

By visual observation, no color change was found in the immobilization section **14.** Thus, the CRP (detection target substance) in the sample solution was not detected.

As described above, a detection target substance in a sample solution can be correctly measured by using an immunochromatography test strip of the present invention even when a sample solution is handled using a blood tube which contains a substance having a chelating effect.

### Industrial Applicability

The present invention is useful for the fields of environmental measurement, food management and medical diagnosis.

## Claims

1. A chromatography test strip for measuring a detection target substance contained in a sample solution, the chromatography test strip comprising a base material, wherein:
the base material includes a sample introduction section, a labeling section and an immobilization section which are arranged such that the sample solution introduced into the sample introduction section migrates through the labeling section to the immobilization section; and
a metal salt is contained in at least any of the sample introduction section, the labeling section, and an area between the sample introduction section and the labeling section.

2. The chromatography test strip of claim 1, wherein the metal salt exists on the surface of the base material or exists inside the base material.

3. The chromatography test strip of claim 1, wherein:
the labeling section includes a first binding substance which specifically binds to the detection target substance, the first binding substance being labeled with a labeling substance; and
the immobilization section includes a second binding substance which specifically binds to the detection target substance.

4. The chromatography test strip of claim 3, wherein the first binding substance and the second binding substance are antibodies that specifically bind to the detection target substance.

5. The chromatography test strip of claim 1, wherein the metal salt is a salt of a divalent metal ion.

6. The chromatography test strip of claim 5, wherein the divalent metal ion is calcium ion.

7. The chromatography test strip of claim 6, wherein the first binding substance and the second binding substance are an anti-CRP antibody.

8. The chromatography test strip of claim 5, wherein the metal salt is calcium chloride.

9. A chromatography test strip for measuring a detection target substance contained in a sample solution, comprising:
a base material;
a sample introduction layer provided above the base material, the sample introduction layer containing a metal salt;
a labeling layer provided on the base material so as to be in contact with the sample introduction layer, the labeling layer containing a first binding substance which specifically binds to the detection target substance in a state such that the first binding substance can be eluted into the sample solution, the first binding substance being labeled with a labeling substance; and
an immobilization layer provided on the base material so as to be in contact with the labeling layer, the immobilization layer containing a second binding substance which specifically binds to the detection target substance in a state such that the second binding substance is immobilized.

10. The chromatography test strip of claim 9, further comprising an absorption layer provided above the base material so as to be in contact with the immobilization layer for absorbing the sample solution.

11. A production method of a chromatography test strip used for measuring a detection target substance contained in a sample solution, comprising the steps of:
(a) preparing a base sheet;
(b) forming a metal salt supporting region, a labeled antibody region, an immobilized antibody region such that these regions extend in parallel to each other and the labeled antibody region is provided between the metal salt supporting region and the immobilized antibody region; and
(c) cutting the base sheet in a direction perpendicular to the metal salt supporting region, the labeled antibody region and the immobilized antibody region, thereby obtaining base sheet strips,
wherein the metal salt supporting region includes a metal salt supported thereon,
the labeled antibody region includes a first binding substance which specifically binds to the detection target substance in a state such that the first binding substance can be eluted into the sample solution, the first binding substance being labeled with a labeling substance; and
the immobilized antibody region includes a second binding substance which specifically binds to the detection target substance in a state such that the second binding substance is immobilized.
